# EUROPEAN PATENT APPLICATION

(11) **EP 3 842 460 A1**
(43) Date of publication of application: **30.06.2021**
(21) Application number: 20766227.1
(22) Date of filing: 25.02.2020
(51) Int. Cl.: C07K 16/28, C12N 15/13, C12N 15/63, A61K 39/00, A61P 35/00, G01N 33/53

(54) **ANTIBODY FOR HER2 CONCOMITANT DIAGNOSIS IMMUNOHISTOCHEMICAL DETECTION AND APPLICATION THEREOF**

(30) Priority: 01.03.2019 CN 201910155276
(71) Applicant: RemeGen Co., Ltd., Shandong 264006 (CN)
(72) Inventor: YU, Zhanjiao, Yantai, Shandong 264006 (CN); LI, Yuanhao, Yantai, Shandong 264006 (CN); HUANG, Changjiang, Yantai, Shandong 264006 (CN); LIANG, Wang, Yantai, Shandong 264006 (CN); MAO, Haiyan, Yantai, Shandong 264006 (CN); FANG, Jianmin, Yantai, Shandong 264006 (CN)
(74) Representative: HGF
(86) International application number: PCT/CN2020/076510
(87) International publication number: WO 2020/177570

(57) **Abstract**

The invention provides an antibody for human epidermal growth factor receptor 2 (Her2) concomitant diagnostic immunohistochemical detection (IHC). When the antibody is used as a primary antibody for immunohistochemical detection of Her2 expression, false positives in detection results caused by the absence of an extracellular region can be avoided. Moreover, the antibody can still recognize and bind to the corresponding epitope in the detection sample when the patient's immunohistochemical detection sample is not repaired by an antigen (or epitope), thereby reducing the issue of a false negative caused by the difference in repairing methods of the antigen (or epitope) in an IHC test.

## Description

### FIELD

The present disclosure relates to the field of companion diagnostic of biological drugs, in particular to a detection antibody for companion diagnostic of drugs targeting Her2 (human epidermal growth factor receptor 2) and the detection application and method thereof.

### BACKGROUND

Cancer is the main disease that harms human health today. In China, the incidence of cancer is rising rapidly. An increasing number of evidence shows that cancers are a group of complex and diverse diseases, and the patients may exhibit similar symptoms and have the same pathological changes, but they may be caused by completely different genetic changes. Because of this heterogeneity, the response rate of cancer patients with the same type of pathology to the currently available drugs varies considerably. Only a part of cancer patients respond to a particular treatment. Since it is impossible to judge the sensitivity and resistance of the individuals with different tumor to drugs before treatment, many patients often suffer from unnecessary and (or) very damaging (side effects) treatment. The current theoretical and practical basis for supporting individualized or precision medicine is the molecular genetic difference between individuals, which is considered to be the decisive factor for human disease susceptibility and drug response. An early example fully demonstrated the relationship between genetics and precision medicine of tumors: at the time of the targeted anti-cancer drugs gefitinib (Iressa) and erlotinib (Tarceva) were initially marketed, the side effects of drugs used in lung carcinoma patients were much improved compared with those in chemotherapy, but the drug efficacy was not very significant, and thus they were only used as second-line drugs for lung carcinoma treatment. However, it was later found that gefitinib and erlotinib had excellent therapeutic effects in lung carcinoma patients with EGFR specific gene mutations, and have now become the first-line standard treatment for clinical treatment of such patients. This proves the relevance of tumor gene mutations to drugs and treatment responses, and it is the general direction of precision medicine to determine the targeted treatment regimen of patients according to the information and combination of gene mutations (Reference 1: Precision Medicine for Tumors: Concept, Technology and Perspectives, Hang Bo, et al., Science & Technology Review, Volume 33, Number 15, Pages 14-21, 2015).

Companion diagnostics (CDx) is an *in vitro* diagnostic technology related to targeted drugs, mainly by measuring the expression levels of the proteins and variant genes in the human body to understand the therapeutic response of different patients to specific drugs, screen out the most suitable drug users and treat them with targeted individualized treatment, so as to improve their treatment prognosis and reduce the cost of health care. The US FDA issued the "Companion Diagnostic Guidelines" on August 06, 2014. Companion diagnostic helps to determine the patient population most likely to respond to therapeutic drugs, promote the use of drugs in a relatively limited market, and improve the effectiveness and safety of drugs. In the development of drugs, CDx facilitates the design of clinical trial scheme with small samples to achieve more clear and definite results with less investment in the development process. The advantage of companion diagnostic is that it can screen out effective treatment regimens for patients, save the time and cost of ineffective treatment, improve compliance of patients with medication, reduce the incidence of adverse reactions, and ensure the safety and efficacy of drugs (Reference 2: Companion diagnostic - Individualized treatment booster, Thomson Reuters, Progress in Pharmaceutical Sciences, Volume 39, Number 6, Pages 463-477, 2015).

Her2, also known as ErbB2, is the second member of the EGFR family and causes the activation of the EGFR signaling pathway by forming a heterodimer with three other members of the EGFR family. The activation of the EGFR signaling pathway is usually associated with the abnormal proliferation of cells and tumorigenesis, so Her 2 has become one of the therapeutic targets of various cancers (such as breast carcinoma, gastric carcinoma, gastroesophageal carcinoma, oesophageal carcinoma, ovarian carcinoma, endometrial carcinoma, lung carcinoma, urothelial carcinoma, bladder carcinoma, etc.; see Reference 16: Human Epidermal Growth Factor Receptor 2 (HER2) in Cancers: Overexpression and Therapeutic Implications, Nida Iqbal and Naveed Iqbal, Molecular Biology International, Volume 2014, Article ID 852748). At present, a variety of therapeutic drugs targeting Her2 including mazumab and pertuzumab have been marketed or are in clinical stage. The increase in Her-2 activity is generally thought to be related to the Her2 gene amplification, the up-regulation of Her2 protein expression, and the mutations of the Her-2 protein. Among them, the HER2 gene amplification and the resulting up-regulation of the Her-2 protein expression are the most common. Among the 34 companion diagnostic reagents approved by US FDA (by the end of February 2019) (Reference 3: https://www.fda.gov/MedicalDevices/ProductsandMedicalProcedures/InVitroDiagnostics/ucm30 1431.htm), it shows that since 1998, there have been 9 approved companion diagnostic kit products involving drugs targeting Her2 (Reference 4: HER2 testing: Current status and future directions, Edith A. Perez et al., Cancer Treatment Reviews, Volume 40, Pages 276-284, 2014) (see Table 1). This indicates a continuing market demand for companion diagnostic reagents for this targeted drug.

**Table 1 FDA approved companion diagnostic kits before HER-2 targeted therapy (Reference 4)**

| Serial Number | Detection Method | Trade Name | Manufactur er | FDA Approval Date | Target Indications and Targeted Drugs |
|---|---|---|---|---|---|
| 1 | Semi-Quantitative IHC | HercepTest | DAKO | September 1998 | Breast carcinoma Herceptin (trastuzumab) Perjeta (pertuzumab) Kadcyla (ado-trastuzumab emtansine) Gastric carcinoma and gastroesophageal carcinoma Herceptin (trastuzumab) |
| 2 | IHC | PATHWAY-anti-Her2/neu (4B5) Rabbit Monoclonal Primary Antibody | Ventana Medical Systems Inc | November 2000 | Breast carcinoma Herceptin (trastuzumab) |
| 3 | IHC | InSite Her-2/neu KIT | Biogenex Laboratorie s Inc | December 2004 | Breast carcinoma Herceptin (trastuzumab) |
| 4 | Semi-quantitative IHC | Bond Oracle HER2 IHC System | Leica Biosystems | April 2012 | Breast carcinoma Herceptin (trastuzumab) |
| 5 | FISH | PathVysion | Abbott Molecular Inc | December 2001 | Breast carcinoma Herceptin (trastuzumab) |
| 6 | FISH | HER2 FISH pharmDx Kit | DAKO | May 2005 | Breast carcinoma Herceptin (trastuzumab) Perjeta (pertuzumab) Kadcyla (ado-trastuzumab emtansine) Gastric carcinoma and gastroesophageal carcinoma Herceptin (trastuzumab) |
| 7 | CISH | SPOT-LIGH THER2 CISH Kit | Life Technologie s Inc | July 2008 | Breast carcinoma Herceptin (trastuzumab) |
| 8 | CISH | INFORM HER2 dual ISH DNA probe cocktail | Ventana Medical Systems Inc | June 2011 | Breast carcinoma Herceptin (trastuzumab) |
| 9 | CISH | HER2 CISH pharmDx Kit | DAKO | November 2011 | Breast carcinoma Herceptin (trastuzumab) |

| | | | | | |
|---|---|---|---|---|---|
| Note: CISH, chromogenic in situ hybridization; FISH, fluorescence in situ hybridization; HER2, human epidermal growth factor receptor 2; IHC, immunohistochemistry. | | | | | |

At present, among the companion diagnostic kits approved by the US FDA for drugs targeting Her2, there are four types based on immunohistochemical detection (two are semi-quantitative IHC and two are IHC), and five types based on in situ hybridization (two are FISH and three are CISH). Immunohistochemistry (IHC) detects Her2 protein expression on the cell membrane, and in situ hybridization (ISH) detects Her2 gene amplification. The principles and characteristics of the three methods used for detection are shown in Table 2.

**Table 2 The detection principles and characteristics of IHC, CISH and FISH**

| Method Name | Principles and Characteristics |
|---|---|
| IHC (immunohistochemistry) | The primary antibody is used to specifically recognize the antigen in the tissue, and then the horseradish peroxidase is used to label the secondary antibody to recognize the primary antibody. The color is developed by catalyzing DAB color development agent with thorseradish peroxidase and finally observed under an optical microscope to realize the localization and qualitative study of antigens in tissues. |
| | Characteristics: Low cost and easy operation |
| CISH (chromogenic in situ hybridization) | The gene probe is labeled with digoxin or biotin to identify the target sequence, and then the anti-digoxin or -biotin antibody labeled with peroxidase or alkaline phosphatase is used to recognize the probe. Finally, the color is developed by catalyzing the substrate with peroxidase or alkaline phosphatase to achieve the purpose of observing chromosome changes under ordinary light microscope. |
| | Characteristics: simple and economical, lower cost than FISH |
| FISH (fluorescence in situ hybridization) | The fluorescein-labeled gene probes are used to in situ identify oncogene/nucleic acid sequences, and then the changes of chromosomes or microchromosomes were observed under a |
| | fluorescent microscope. |
| | Characteristics: high sensitivity and specificity |

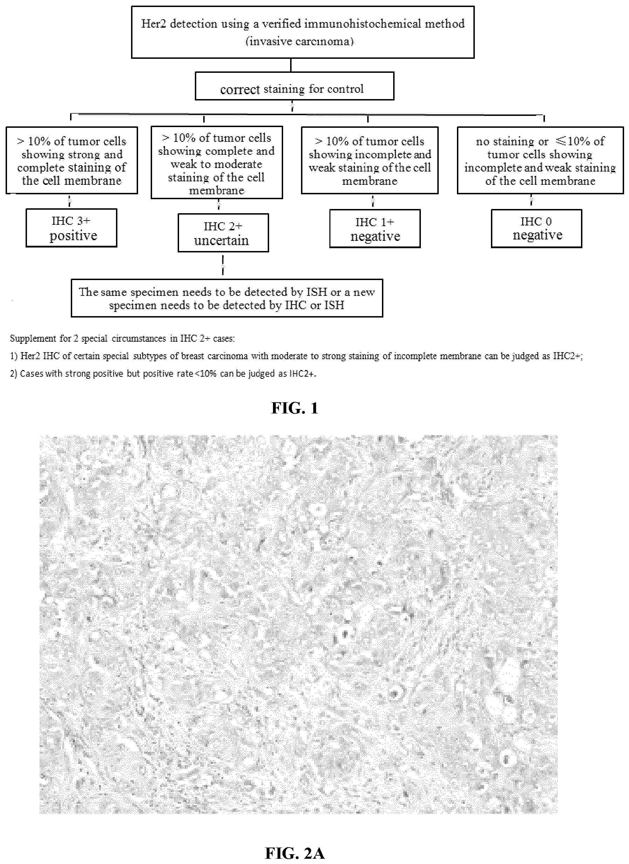

Although the detection results of CISH and FISH have higher accuracy than IHC, the IHC method is superior to the CISH method as a preliminary screening tool, because the IHC method is not only economical (according to an evaluation report made by McGill University Health Centre (MUHC) in 2006, which gives an approximate price comparison between IHC and FISH: the price of a single IHC detection is about US $85 and FISH is about US $381 (Reference 5: https://muhc.ca/sites/defaull/files/micro/m-TAU/HER2reportdraft4finalMay2006.pdf, Page 15); Seema Jabbar et al. (Reference 6: Comparison of Two FDA-Approved Her2 Immunohistochemical Assays for Breast Carcinoma: HercepTest and Pathway Her2 (4B5), Am J Clin Pathol 2018;149:S93-S94) detected a total of 95 samples, and the total cost of using FISH detection was US $5066 (25 samples) and US $14590 (72 samples), with an average of US $202 per detection, while the cost of FISH detection in the above regions is about 3-4 times the cost of IHC detection; furthermore, according to the actual detecting costs of hospitals in China at the current stage, FISH costs about 1200 yuan, IHC costs about 150 yuan, and FISH costs reach 8 times that of IHC) and fast, but also has a morphological basis for reading, especially it is easy to handle for a large number of samples, with high sensitivity, and relatively high repeatability within and between laboratories. Pathologists usually use the IHC method as the first detection method for condition evaluation. However, there will be false positive results in 2+ cases that are judged to be positive based on the IHC method. The current Her2 detection process recommended by ASCO/CAP (American society of clinical oncology/College of American Pathologists) for breast carcinoma is as follows: the IHC method is firstly used for detecting; cases with an IHC detection result of 3+ are also often positive for FISH, and of 1+ and below are often negative for FISH; and cases with an IHC detection result of 2+ need to be reconfirmed by the FISH method (see Figure 1 for details, Reference 7: Human Epidermal Growth Factor Receptor 2 Testing in Breast Cancer, American Society of Clinical Oncology College of American Pathologists Clinical Practice Guideline Focused Update, Antonio C. Wolff et al., Arch Pathol Lab Med, Volume 142, Pages 1364-1382, November 2018).

The false positive problem in the IHC method in the prior art: at present, the initial detection of the Her2 expression state of the patient sample mainly depends on the IHC detection. In the IHC detection, screening a primary antibody with high effectiveness and reliability is a particularly critical step. The antibody in the IHC detection fundamentally determines the sensitivity or specificity of the entire detection result. Prescott et al. pointed out that 42.1% of the diagnostic differences in IHC detection were caused by poor antibodies (Reference 8: Immunohistochemistry as an Important Tool in Biomarkers Detection and Clinical Practice, Leandro Luongo de Matos et al., Biomarker Insights, Volume 5, Pages 9-20, 2010; Reference 9: Audit of tumour histopathology reviewed by a regional oncology centre. Prescott RJ et al. J Clin Pathol. Volume 48, Pages 245-249. 1995). Currently, the commonly used antibodies for detection of the Her2 expression state mainly include 4B5 clone of Roche, CB11 clone of Leica and the like. The above detection antibodies all recognize the intracellular region of Her2 molecule, and all therapeutic antibody drugs targeting Her2 recognize the extracellular region of the Her2 molecule. In view of the Her2 molecule has a mutant form in which the extracellular region is deleted (95-100 kDa p95HER2 (648-CTF), 100-115 kDa p95HER2 (611-CTF)) (Reference 10: p95HER2 and breast cancer. Arribas J. et al. Cancer Res. Volume 71, Number 5, Pages 1515-1519, 2011), the corresponding cases are unresponsive or insensitive to drugs such as trastuzumab and pertuzumab that target the extracellular region of Her2, however, this part of the case will show false positives by using the above commercial companion diagnostic reagents to detect, which is also a major reason why the response rate of antibody-based Her-2 targeted drugs currently accepted is only about 60%. Therefore, the development of a detection antibody that specifically recognizes the extracellular region of the Her2 molecule and a companion diagnostic IHC reagent product developed based on the corresponding antibody will effectively reduce the false positive problems that currently occur in the companion detection of her2, thereby saving the time of ineffective treatment and huge cost for the corresponding patients.

The false negative problem in the IHC method in the prior art: in immunohistochemical detection, correct antigen (or epitope) retrieval is a key step in the accuracy of immunohistochemical detection results. Due to the cross-linking of formaldehyde and protein to form aldehyde cross-linked protein during tissue fixation, this compound blocks the antigen, resulting in a decrease in the specific response of the antibody to the antigen, resulting in false negatives. The fully automatic multifunctional histopathological detection system has the advantages of uniform dyeing and coloring, accurate positioning, and strong repeatability. However, the equipment is expensive, the cost of reagents is high, and the scope of use is limited. The manual operation method is still commonly used in the pathology department of primary hospitals. At present, the retrieval methods and retrieval solutions used in various departments in the manual operations are different. Even if the retrieval method is the same, the details of the retrieval are very different. However, correct retrieval is very important for the expression of antibodies. Therefore, the problems that need to be solved in the current IHC detection is also to improve the repeatability of the operation results of different primary hospital staff and how to avoid the false negative problem generated during the retrieval process (Reference 11: Optimum Conditions for Manual Operation of Citrate High Temperature and High Pressure Antigen Retrieval in Breast Carcinoma Her-2 Immunohistochemical, Zhu Guoliang, et al., Chinese Journal of Clinical and Experimental Pathology, Volume 33, Number 2, Pages 219-220; Reference 12: Comparison of the effects of different antigen retrieval solutions on immunohistochemical results, Wang Qi et al, Chinese Journal of Laboratory Diagnosis, Volume 19, Number 8, Pages 1246-1248, August 2018; Reference 13: Advanced Course in Pathology, edited by Lai Maode, published: Beijing: People's Military Medical Press, 2013.03;ISBN Number: 978-7-5091-6426-6, Page 856).

Among the drugs targeting Her2, WO2015074528A1 and CN105008398A disclose an antibody-drug conjugate (i.e., antibody-drug conjugate, ADC) comprising an antibody capable of specifically binding Her2, wherein the antibody is conjugated with one or more therapeutic agents selected from the group consisting of MMAE and MMAF, and the antibody comprises a heavy chain and a light chain, wherein (i) the amino acid sequences of the CDRs 1-3 of the heavy chain region are DYYIH, RVNPDHGDSYYNQKFKD and ARNYLFDHW, respectively; and (ii) the amino acid sequences of the CDRs 1-3 of the light chain are KASQDVGTAVA, WASIRHT and HQFATYT, respectively. Or further, the antibody is derived from an antibody secreted by the hybridomas deposited in the China General Microbiological Culture Collection Center with the depositary number CGMCC No. 8102 on August 22, 2013. Or further, the antibody is derived from an antibody secreted by the CHO cells deposited in the China Center for Type Culture Collection with the depositary number CCTCC C2013170 on November 06, 2013. The conjugate comprising an antibody capable of specifically binding HER2 is used to treat patients with Her2-positive cancer, which includes breast carcinoma, ovariancarcinoma or gastriccarcinoma, or further is lapatinib and/or Herceptin-resistant breast carcinoma, ovariancarcinoma or gastriccarcinoma. In order to ensure the high correspondence between the results of IHC detection antibody and the treatment of the conjugate, there is also a great urgency to develop the matched companion diagnostic antibody detection product of the conjugate.

### SUMMARY

In order to solve the above problems, the present disclosure provides a detection antibody targeting the extracellular region of Her2, which specifically binds to the extracellular domain IV region of Her2 protein.

An exemplary amino acid sequence of Her2 is shown in NCBI GenBank ID: AAA75493.1, and the corresponding extracellular domain IV region of the protein is located at amino acids 511-643. Johan Rockberg et al. (Reference 14: Discovery of epitopes for targeting the human epidermal growth factor receptor 2 (HER2) with antibodies, Johan Rockberg et al., MOLECULAR ONCOLOGY, Volume 3, Pages 238-247, 2009) and the "Her2/ERBB2 Protein, Human, Recombinant (ECD, domain IV, His Tag)" (Polyhistidine tag-linked human ERBB2 (AAA75493. 1) (Pro489-Cys630) region) (Cat: 10004-H08H4, https://www.sinobiological.com/Human-HER2-ErbB2-Protein-ECD-domain-IV-His-Tag-p22311 9.html) (characteristic description: the binding ability was detected by functional ELISA; and it can bind to herceptin (the EC50 value thereof is 10-40 ng/mL)) marketed by Beijing Yiqiao Shenzhou Technology Co., Ltd. (Sino Biological) proves that the extracellular domain IV region of Her2 protein can be used as an independent immunogen and still remains the spatial configuration of the corresponding area when in the complete Her2.

The prior art discloses an antibody with the following CDR combinations targeting the extracellular domain IV of Her2 protein: (i) the CDRs 1-3 of the heavy chain variable regions are GFNIKDTYIH, RIYPTNGYTRYADSVKG and WGGDGFYAMDV, respectively; and the CDRs 1-3 of the light chain variable region are RASQDVNTAVAW, SASFLES and QQHYTTPPT, respectively (WO9222653A1); and (ii) the CDRs 1-3 of the heavy chain variable regions are GFNIKDTYIH, RIYPTNGYTRYADSVKG and WGGDGFYAMDY, respectively; and the CDRs 1-3 of the light chain variable region are RASQDVNTAVA, SASFLES and QQHYTTPPT, respectively; (Reference 15: Molecular dynamic simulation of Trastuzumab F (ab') 2 structure in corporation with HER2 as a theranostic agent of breast cancer, S Hermanto et al., Journal of Physics: Conference Series, Volume 835, Number 1, Pages 1-11, 2017). The use of such antibodies is all therapeutic antibodies.

The present disclosure has surprisingly found that some antibodies that specifically bind to the extracellular domain IV region of Her2 protein can be used as primary antibodies in immunohistochemical detection. Without performing antigen (or epitope) retrieval treatment on the patient's immunohistochemical detection sample, these antibodies can still be specifically and effectively recognized and detected, effectively reducing the false negative problems due to the differences between antigen (or epitope) retrieval treatment methods in IHC detection. At the same time, since the above retrieval treatment steps are not required, the working intensity can be effectively reduced, the detection time can be shortened, and thus the efficiency of IHC detection can be improved.

In addition, the present disclosure has also surprisingly found that, an antibody that binds to the same epitope (located in the extracellular domain IV region of Her2 protein) as the antibody (the amino acid sequences of the CDRs 1-3 of the heavy chain region are DYYIH, RVNPDHGDSYYNQKFKD and ARNYLFDHW, respectively; and the amino acid sequences of the CDRs 1-3 of the light chain are KASQDVGTAVA, WASIRHT and HQFATYT, respectively) in the antibody (RC48) drug conjugate (RC48-ADC) targeting Her2 disclosed in WO2015074528A1 and CN105008398A or that has the same light chain and heavy chain CDRs above can be used as a companion diagnostic IHC antibody to determine Her2 positive. Because the epitope is located in the extracellular domain IV region of Her2 protein, the detection of the corresponding IHC antibody avoids the possibility of detecting the mutant form in which the extracellular region of the Her2 molecule is deleted as positive, so it also effectively solves the problem of false positive caused by the detection of the primary antibody against the intracellular region of Her2. At the same time, because it is the same as the CDR region of the antibody in the targeted drug, the epitope bound during the detection is exactly the same as the targeted epitope of the therapeutic drug. This high correspondence between the binding epitope of the detection antibody and that of the pharmaceutical antibody highly guarantees the pertinence and effectiveness of the targeted drug when used.

The technical solution of the present disclosure is as follows:
The present disclosure provides an antibody for companion diagnostic immunohistochemical (IHC) detection of human epidermal growth factor receptor 2 (Her2), wherein the antibody specifically binds to the extracellular domain IV region of Her2 protein and has the capability of effectively detecting IHC samples without antigen or epitope retrieval treatment.

Further, the Fc fragment of the antibody is the Fc fragment of a non-human mammal antibody, preferably a murine-derived Fc fragment or a rabbit-derived Fc fragment.

Further, the antibody competitively binds to the same or similar epitope with a CDR-defined antibody, wherein the CDRs 1-3 of the heavy chain variable region of the CDR-defined antibody have the amino acid sequences shown in SEQ ID NOs: 1-3, respectively; and the CDRs 1-3 of the light chain variable region of the CDR-defined antibody have the amino acid sequences shown in SEQ ID NOs: 4-6, respectively.

Further, in the antibody:
(i) the CDR1 of the heavy chain variable region has the amino acid sequence shown in SEQ ID NO: 1 or the amino acid sequence obtained after 1 or 2 amino acids substitution on SEQ ID NO: 1; or/and the CDR2 of the heavy chain variable region has the amino acid sequence shown in SEQ ID NO: 2 or the amino acid sequence obtained after 1, 2, 3, 4 or 5 amino acids substitution on SEQ ID NO: 2; or/and the CDR3 of the heavy chain variable region has the amino acid sequence shown in SEQ ID NO: 3 or the amino acid sequence obtained after 1, 2 or 3 amino acids substitution on SEQ ID NO : 3; and/or
(ii) the CDR1 of the light chain variable region has the amino acid sequence shown in SEQ ID NO: 4 or the amino acid sequence obtained after 1, 2, 3, or 4 amino acids substitution on SEQ ID NO: 4; or/and the CDR2 of the light chain variable region has the amino acid sequence shown in SEQ ID NO: 5 or the amino acid sequence obtained after 1 or 2 amino acids substitution on SEQ ID NO: 5; or/and the CDR3 of the light chain variable region has the amino acid sequence shown in SEQ ID NO: 6 or the amino acid sequence obtained after 1 or 2 amino acids substitution on SEQ ID NO: 6;
   and/or
(iii) the immunoglobulin Fc fragment is a murine-derived IgG Fc fragment, or further is a murine-derived IgG1 Fc fragment.

Further, in the antibody:
(i) the CDRs 1-3 of the heavy chain variable region have the amino acid sequences shown in SEQ ID NOs: 1-3, respectively; and
(ii) the CDRs 1-3 of the light chain variable region have the amino acid sequences shown in SEQ ID NOs: 4-6, respectively.

Further, the antibody:
(i) the heavy chain of the antibody comprises the amino acid sequence shown in SEQ ID NO: 7 or an amino acid sequence having at least 80% sequence identity thereto; and/or
(ii) the light chain of the antibody comprises the amino acid sequence shown in SEQ ID NO: 8 or an amino acid sequence having at least 80% sequence identity thereto; and
(iii) the CDRs 1-3 of the heavy chain variable region of the antibody have the amino acid sequences shown in SEQ ID NOs: 1-3, respectively; the CDRs 1-3 of the light chain variable region of the antibody have the amino acid sequences shown in SEQ ID NOs: 4-6, respectively.

Further, an antibody R48M is provided, and the amino acid sequence thereof is as follows:
1) Heavy chain (Hc) 
EVQLVQSGAEVKKPGATVKISCKVSGYTFTDYYIHWVQQAPGKGLEWMGRVNPDHGDS
YYNQKFKDKATITADKSTDTAYMELSSLRSEDTAVYFCARNYLFDHWGQGTLVTVSSAK
TTPPSVYPLAPGSAAQTNSMVTLGCLVKGYFPEPVTVTWNSGSLSSGVHTFPAVLQSDLY
TLSSSVTVPSSTWPSETVTCNVAHPASSTKVDKKIVPRDCGCKPCICTVPEVSSVFIFPPKP
KDVLTITLTPKVTCVVVDISKDDPEVQFSWFVDDVEVHTAQTQPREEQFNSTFRSVSELPI
MHQDWLNGKEFKCRVNSAAFPAPIEKTISKTKGRPKAPQVYTIPPPKEQMAKDKVSLTC
MITDFFPEDITVEWQWNGQPAENYKNTQPIMDTDGSYFVYSKLNVQKSNWEAGNTFTC
SVLHEGLHNHHTEKSLSHSPGI（SEQ ID NO: 7）
2) Light chain (Lc)

DIQMTQSPSSVSASVGDRVTITCKASQDVGTAVAWYQQKPGKAPKLLIYWASIRHTGVPS
RFSGSGSGTDFTLTISSLQPEDFATYYCHQFATYTFGGGTKVEIKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC（SEQ ID NO: 8）

The present disclosure further provides a nucleic acid molecule encoding the above antibody.

The present disclosure further provides a vector comprising the above nucleic acid molecule.

The present disclosure further provides the use of the above antibody in the manufacture of a Her2 companion diagnostic immunohistochemical detection product, and the immunohistochemical detection product has the function of not requiring the step of the antigen or epitope retrieval during the immunohistochemical detection.

The present disclosure further provides a Her2 companion diagnostic immunohistochemical detection kit comprising the above antibody, and the kit has the function of not requiring the step of the antigen or epitope retrieval during the immunohistochemical detection.

The present disclosure further provides a Her2 companion diagnostic immunohistochemical detection method, which uses the above antibody as a primary antibody for the detection. Or further, there is no antigen or epitope retrieval step during the immunohistochemical detection.

The indications targeted by the Her2 companion diagnostic described in the present disclosure are Her2-related cancers, and further preferably breast carcinoma, gastric carcinoma, gastroesophageal carcinoma, oesophageal carcinoma, ovarian carcinoma, endometrial carcinoma, lung carcinoma, urothelial carcinoma, or bladder carcinoma. (see Reference 16)

The advantageous effects brought by the technical solution of the present disclosure are as follows: the present disclosure provides an antibody capable of targeting the extracellular domain IV region of Her2 protein. When using the antibody as a primary antibody to detect the Her2 expression in a sample, false positive results due to the deletion of the extracellular region can be effectively avoided. In addition, in the process of immunohistochemical detection, since the above retrieval treatment steps are not required, the Her2 detection antibody provided by the present disclosure effectively reduces the false negative problems due to the differences between antigen (or epitope) retrieval treatment methods in IHC detection. At the same time, it can effectively reduce the working intensity and shorten the detection time, thereby improving the efficiency of IHC detection.

### BRIEF DESCRIPTION OF DRAWINGS

Figure 1. Guidelines for Her2 detection using a verified immunohistochemistry method;
Figure 2A shows the results of immunohistochemical staining of the case of serial number 1 in Table 7 with RC48M antibody as the primary antibody;
FIG. 2B shows the results of immunohistochemical staining of the case of serial number 1 in Table 7 with 4B5 antibody as the primary antibody;
Figure 3A shows the results of immunohistochemical staining of the case of serial number 2 in Table 7 with RC48M antibody as the primary antibody;
Figure 3B shows the results of immunohistochemical staining of the case of serial number 2 in Table 7 with 4B5 antibody as the primary antibody;
Figure 4A shows the results of immunohistochemical staining of the case of serial number 3 in Table 7 with RC48M antibody as the primary antibody;
FIG. 4B shows the results of immunohistochemical staining of the case of serial number 3 in Table 7 with 4B5 antibody as the primary antibody;
Figure 5A shows the results of immunohistochemical staining of the case of serial number 4 in Table 7 with RC48M antibody as the primary antibody;
FIG. 5B shows the results of immunohistochemical staining of the case of serial number 4 in Table 7 with 4B5 antibody as the primary antibody;
Figure 6A shows the results of immunohistochemical staining of the case of serial number 5 in Table 7 with RC48M antibody as the primary antibody;
FIG. 6B shows the results of immunohistochemical staining of the case of serial number 5 in Table 7 with 4B5 antibody as the primary antibody;
Figure 7A shows the results of immunohistochemical staining of the case of serial number 6 in Table 7 with RC48M antibody as the primary antibody;
FIG. 7B shows the results of immunohistochemical staining of the case of serial number 6 in Table 7 with 4B5 antibody as the primary antibody;
FIG. 8A shows the results of immunohistochemical staining of the case of serial number 7 in Table 7 with RC48M antibody as the primary antibody;
FIG. 8B shows the results of immunohistochemical staining of the case of serial number 7 in Table 7 with 4B5 antibody as the primary antibody;
Figure 9 shows the structural formula of an RC48-ADC drug, wherein RC48 represents the RC48 antibody.

### DETAILED DESCRIPTION

### DEFINITION

Unless otherwise defined, all terms used herein have the same meaning as understood by those of ordinary skill in the art. For definitions and terms in the art, professionals can in particular refer to Current Protocols in Molecular Biology (Ausubel). The abbreviation for amino acid residues is a standard 3-letter and/or 1-letter code used in the art to refer to one of the 20 commonly used L-amino acids.

As used herein, "antibody" is used in the broadest scope and encompasses various antibody structures including, but not limited to, a monoclonal antibody, a polyclonal antibody, a multispecific antibody (e.g., bispecific antibody), and an antibody fragment. In particular, "antibody" as used herein refers to a protein comprising at least two heavy chains and two light chains interconnected by disulfide bonds. Each heavy chain comprises a heavy chain variable region (one-fifth or one-quarter region on the heavy chain near the N-terminal) and a heavy chain constant region (three-quarters or four-fifths region on the heavy chain near the C-terminal). Each light chain comprises a light chain variable region (a half region on the light chain near the N-terminal) and a light chain constant region (a half region on the light chain near the C-terminal). The variable region of the heavy chain and the variable region of the light chain can be further subdivided into multiple regions with high variability, which is called a complementary determining region (CDR). The "CDR" refers to the hypervariable regions of the heavy and light chains of immunoglobulins, including those determined by the Kabat, Chothia, or IMGT systems. There are three heavy chain CDRs and three light chain CDRs per antibody. Depending on the circumstances, the term CDR as used herein is intended to indicate one or several or even all of these regions, which comprise most of the amino acid residues responsible for binding through the affinity of an antibody to an antigen or a recognition epitope thereof.

The term "Her2 detection antibody" as used in the present disclosure refers to an antibody capable of detecting the expression state of Her2 in a case and capable of binding to Her2. In immunohistochemistry, Her2 detection antibody is used as a primary antibody to detect the expression state of Her2. The "Her2 detection antibody" involved in the present disclosure is capable of targeting the extracellular domain IV region of Her2 protein.

The term "RC48M antibody" as used in the present disclosure refers to an antibody in which the amino acid sequence of the heavy chain variable region is shown in SEQ ID NO: 7, and the amino acid sequence of the light chain variable region is shown in SEQ ID NO: 8.

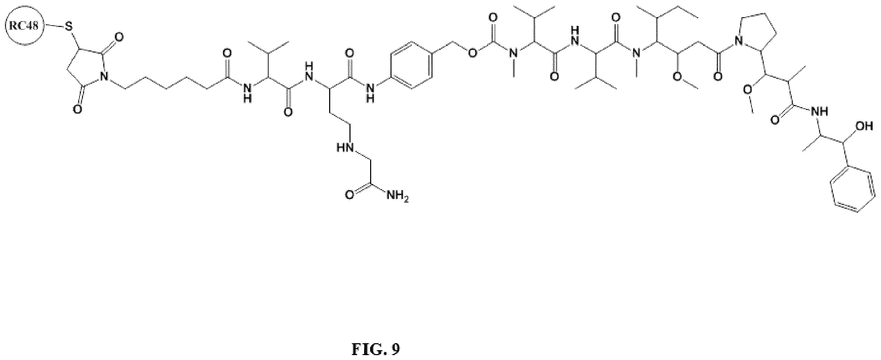

The term "RC48-ADC" or "RC48-ADC drug" or "RC48-ADC targeted drug" as used in the present disclosure refers to an antibody-drug conjugate (i.e. antibody-drug conjugate, ADC) comprising an antibody capable of specifically binding Her2 disclosed in patents WO2015074528A1 and CN105008398A, wherein the antibody is called "RC48 antibody", and (i) the amino acid sequences of the CDRs 1-3 of its heavy chain region are DYYIH, RVNPDHGDSYYNQKFKD and ARNYLFDHW, respectively; and (ii) the amino acid sequences of the CDRs 1-3 of its light chain are KASQDVGTAVA, WASIRHT and HQFATYT, respectively. Or further, the antibody is derived from an antibody secreted by the hybridomas deposited in the China General Microbiological Culture Collection Center with the depositary number CGMCC No. 8102 on August 22, 2013. Or further, the antibody is derived from an antibody secreted by the CHO cells deposited in the China Center for Type Culture Collection with the depositary number CCTCC C2013170 on November 06, 2013. The structure of an RC48-ADC drug is shown in Figure 9.

### EXAMPLES

The present disclosure will be further described from examples in the following. It should be noted that the following examples are further illustrations and explanations of the present disclosure, and should not be considered as limiting the present disclosure.

In the following examples, the RC48M antibody was used as an example of an IHC detection antibody targeting the extracellular domain IV region of Her2 protein. The amino acid sequence of the heavy chain variable region of the RC48M antibody is shown in SEQ ID NO: 7, and the amino acid sequence of the light chain variable region of the RC48M antibody is shown in SEQ ID NO: 8. The detection of breast carcinoma samples was used as an example.

Example 1 Comparison of the overall agreement rate between the IHC detection of RC48M antibody (primary antibody) and the IHC detection of PATHWAY - anti-Her2/neu (4B5) Rabbit Monoclonal Primary Antibody (hereinafter referred to as 4B5 antibody) and the problem of false negative

Comparative detection and analysis of 49 invasive breast carcinoma samples.
1) For 4B5 antibody, follow the product instruction method;
2) Main procedure steps of RC48M IHC detection

**Table 3 Main procedure steps of RC48M IHC detection**

| Step | Procedure and Parameter Description |
|---|---|
| Primary antibody (dilution ratio) | RC48M (1:500) |
| Incubation conditions and time for the primary antibody | Room temperature (18∼20°C), 20 min |
| Antigen retrieval conditions | without retrieval |
| Detection system for the secondary antibody | Polymer Refine Detection |
| Color development time of DAB | 5 min |

See Table 4 for a list of detection results.

**Table 4 List of detection results from 49 samples**

| Number | Tissue Type | Pathology Number | 4B5 Antibody | RC48M Antibody |
|---|---|---|---|---|
| 1 | breast carcinoma | 18-04155-1 | 3+ | 3+ |
| 2 | breast carcinoma | 18-05735-1 | 3+ | 3+ |
| 3 | breast carcinoma | 18-05736-2 | 3+ | 3+ |
| 4 | breast carcinoma | 18-06464-1 | 3+ | 3+ |
| 5 | breast carcinoma | 18-07814-1 | 3+ | 3+ |
| 6 | breast carcinoma | 18-08167-1 | 3+ | 3+ |
| 7 | breast carcinoma | 18-08709-1 | 3+ | 3+ |
| 8 | breast carcinoma | 18-09022-1 | 3+ | 3+ |
| 9 | breast carcinoma | 18-07600-2 | 3+ | 2+ |
| 10 | breast carcinoma | 18-09839-1 | 3+ | 2+ |
| 11 | breast carcinoma | 18-09840-1 | 3+ | 2+ |
| 12 | breast carcinoma | 18-07150-1 | 2+ | 2+ |
| 13 | breast carcinoma | 18-07607-5 | 1+ | 2+ |
| 14 | breast carcinoma | 18-08555-1 | 1+ | 2+ |
| 15 | breast carcinoma | 18-08929-1 | 1+ | 2+ |
| 16 | breast carcinoma | 18-09503-1 | 1+ | 2+ |
| 17 | breast carcinoma | 18-05833-1 | 1+ | 1+ |
| 18 | breast carcinoma | 18-05899-1 | 1+ | 1+ |
| 19 | breast carcinoma | 18-07234-1 | 1+ | 1+ |
| 20 | breast carcinoma | 18-07384-4 | 1+ | 1+ |
| 21 | breast carcinoma | 18-07817-1 | 1+ | 1+ |
| 22 | breast carcinoma | 18-10298-2 | 1+ | 1+ |
| 23 | breast carcinoma | 18-06079-2 | 1+ | 0 |
| 24 | breast carcinoma | 18-07486-1 | 1+ | 0 |
| 25 | breast carcinoma | 18-07728-2 | 1+ | 0 |
| 26 | breast carcinoma | 18-08047-1 | 1+ | 0 |
| 27 | breast carcinoma | 18-08459-1 | 1+ | 0 |
| 28 | breast carcinoma | 18-09312-1 | 1+ | 0 |
| 29 | breast carcinoma | 18-10091-1 | 1+ | 0 |
| 30 | breast carcinoma | 18-08952-1 | 0 | 2+ |
| 31 | breast carcinoma | 18-08580-7 | 0 | 1+ |
| 32 | breast carcinoma | 18-05734-1 | 0 | 0 |
| 33 | breast carcinoma | 18-05827-1 | 0 | 0 |
| 34 | breast carcinoma | 18-05829-1 | 0 | 0 |
| 35 | breast carcinoma | 18-06431-1 | 0 | 0 |
| 36 | breast carcinoma | 18-06551-3 | 0 | 0 |
| 37 | breast carcinoma | 18-06714 | 0 | 0 |
| 38 | breast carcinoma | 18-06715-3 | 0 | 0 |
| 39 | breast carcinoma | 18-06856-1 | 0 | 0 |
| 40 | breast carcinoma | 18-07599-1 | 0 | 0 |
| 41 | breast carcinoma | 18-07822-1 | 0 | 0 |
| 42 | breast carcinoma | 18-08233-1 | 0 | 0 |
| 43 | breast carcinoma | 18-08345-1 | 0 | 0 |
| 44 | breast carcinoma | 18-08930-1 | 0 | 0 |
| 45 | breast carcinoma | 18-09161-1 | 0 | 0 |
| 46 | breast carcinoma | 18-09361-3 | 0 | 0 |
| 47 | breast carcinoma | 18-09437-1 | 0 | 0 |
| 48 | breast carcinoma | 18-09673-1 | 0 | 0 |
| 49 | breast carcinoma | 18-09674-1 | 0 | 0 |

Note: the grading judgment of the staining results was referred to the rules in the "Guideline for HER2 Detection of Breast carcinoma (2014 Edition)", the same below. The above results were summarized, and the results were shown in Table 5.

**Table 5 Statistical analysis table of detection results**

| Number of Cases | RC48M Antibody | | | | Total |
|---|---|---|---|---|---|
| 4B5 antibody | 0 | 1+ | 2+ | 3+ | |
| 0 | 18 | 1 | 1 | 0 | 20 |
| 1+ | 7 | 6 | 4 | 0 | 17 |
| 2+ | 0 | 0 | 1 | 0 | 1 |
| 3+ | 0 | 0 | 3 | 8 | 11 |
| Total | 25 | 7 | 9 | 8 | 49 |

From the above statistical results, there were 33 cases (18+6+1+8) with the same detection grade, accounting for 67.3% (33/49). From the judgment guidance of the current medical decision, 0 and 1+ were determined as negative, 2+ was suspicious, and 3+ was positive, then a total of 26 cases (18+7+1) + 6 cases + 1 case + 8 cases = 41 cases (accounting for 83.7%). The use of both will lead to the same medical decision. The cases with differences in decisions are summarized in Table 6.

**Table 6 List of samples with differences in medical decisions**

| Number | Tissue Type | Pathology Number | 4B5 Antibody | RC48M Antibody |
|---|---|---|---|---|
| 9 | breast carcinoma | 18-07600-2 | 3+ | 2+ |
| 10 | breast carcinoma | 18-09839-1 | 3+ | 2+ |
| 11 | breast carcinoma | 18-09840-1 | 3+ | 2+ |
| 13 | breast carcinoma | 18-07607-5 | 1+ | 2+ |
| 14 | breast carcinoma | 18-08555-1 | 1+ | 2+ |
| 15 | breast carcinoma | 18-08929-1 | 1+ | 2+ |
| 16 | breast carcinoma | 18-09503-1 | 1+ | 2+ |
| 30 | breast carcinoma | 18-08952-1 | 0 | 2+ |

As mentioned above, Seema Jabbar et al. (Reference 6: Comparison of Two FDA-Approved Her2 Immunohistochemical Assays for Breast Carcinoma: HercepTest and Pathway Her2 (4B5), Am J Clin Pathol 2018; 149: S93-S94) conducted HercepTest and Pathway Her2 (4B5) (referred to as 4B5) detections on a total of 95 breast carcinoma samples. The detection results of HercepTest were 72 suspicious + 23 positive; wherein the 72 suspicious samples were detected by 4B5, and 52 (52/72=72%) were negative. Of the 95 samples detected by 4B5, 52 (55%) were negative, 25 (26%) were suspicious, and 18 (19%) were positive. Confirmation of the results by FISH detection shows that 4B5 has a higher consistency with the results of FISH detection compared to HercepTest detection. In addition, among the 52 negative detected by 4B5, 3 cases were verified as positive by FISH detection, which indicates that there was a certain false negative result in the 4B5 detection. Combining with the results in Table 6, part of the results of the 4B5 detection were interpreted as 2+ for the results of the R48M detection and became suspicious cases, which required a process of the FISH or CISH detection (ISH detection) to determine. Although the use of the RC48M detection resulted in the addition of further ISH confirmation detection steps in some cases, this to a certain extent avoids the problem of partial false negatives in the results of 4B5 detection, thereby avoiding the problem of missing the better treatment period and missed the use of targeted therapeutic drugs to develop targeted therapy due to the problem of false negative detection in some patients. In addition, combining the results of Reference 6 and the above detection data, the consistency of the results of the RC48M detection and the results of the FISH detection should also be higher than the results of the HercepTest detection.

### Example 2 Analysis of false positive problem

A total of 7 phase I clinical samples of RC48-ADC drug were detected in parallel with RC48M antibody and 4B5 for comparison. Seven paraffin sections of breast carcinoma tissue were used in each group. The primary antibody in Group 1 was RC48M antibody and in Group 2 was 4B5 antibody; and the two groups of experiments both used the rabbit and mouse universal immunohistochemical secondary antibody kit (purchased from Fuzhou Maixin Biotechnology Development Co., Ltd.). The specific experimental process is as follows:
(1) Dewaxing and hydration: the slices were baked at 65°C for 2 h, then soaked sequentially as follows: in xylene for 20 min, in absolute ethanol for 5 min, in 95% ethanol for 5 min, and in 75% ethanol for 5 min; and then the slices treated as above were transferred into 0.01 M PBST for use;
(2) Antigen retrieval: the slices of Group 2 were transferred into the sodium citrate buffer and heated at 98°C for 20 min; and the slices of Group 1 did not require antigen retrieval.
(3) Blocking: the slices of Group 1 and Group 2 were blocked with 3% hydrogen peroxide for 10 min, and then soaked twice with 0.01 MPBST for 3 min at a time;
(4) Primary antibody: the slices of Group 1 and Group 2 were placed in a wet box, the RC48M antibody was diluted to 1 µg/ml, and then the diluted RC48M antibody and 4B5 antibody were added dropwise onto the corresponding slices of Group 1 and Group 2, respectively, incubated at 37°C for 60 min, and soaked twice with 0.01 M PBST for 3 min each time after returned to room temperature;
(5) Secondary antibody: the rabbit and mouse universal secondary antibody in the rabbit and mouse universal immunohistochemistry secondary antibody kit was added dropwise onto the slices of Group 1 and Group 2 in an amount of 100 µl/piece, respectively, incubated at 37°C for 20 min, and soaked twice with PBST (0.01 M) for 3 min each time; then the reaction amplifying agent in the above kit was added dropwise onto the slices of Group 1 and Group 2 in an amount of 100 µl/piece, incubated at 37°C for 20 min, and soaked twice with PBST (0.01 M) for 3 min each time;
(6) DAB color development: DAB color development kit (purchased from Fuzhou Maixin Biotechnology Development Co., Ltd.) was employed. 50 µl each of the dilution buffer, DAB chromogen and substrate in 1 mL of the kit were taken out and mixed well, then added onto the slices, color developed at room temperature for 1-3 min, and washed with distilled water;
(7) Hematoxylin counterstaining: 100 µL of hematoxylin staining solution (purchased from Fuzhou Maixin Biotechnology Development Co., Ltd.) was added dropwise to each slice, stained at room temperature for 2-5 min, and washed with tap water for 5 min until return of blue.
(8) Dehydration: the slices of Group 1 and Group 2 were soaked sequentially as follows: in 75% ethanol for 5 min, in 95% ethanol for 5 min, in 100% ethanol for 5 min, and in xylene for 10 min; and then the slices were taken out and xylene was volatilized.
(9) Mounting.

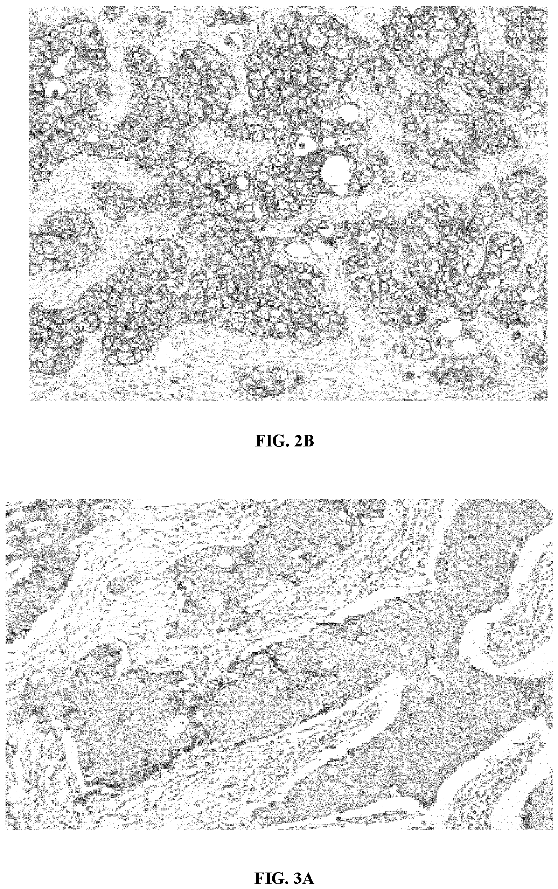

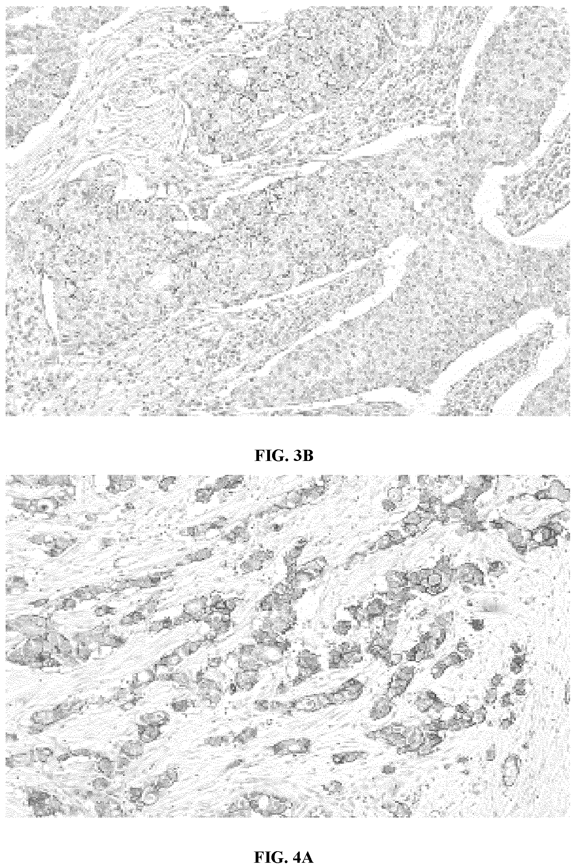

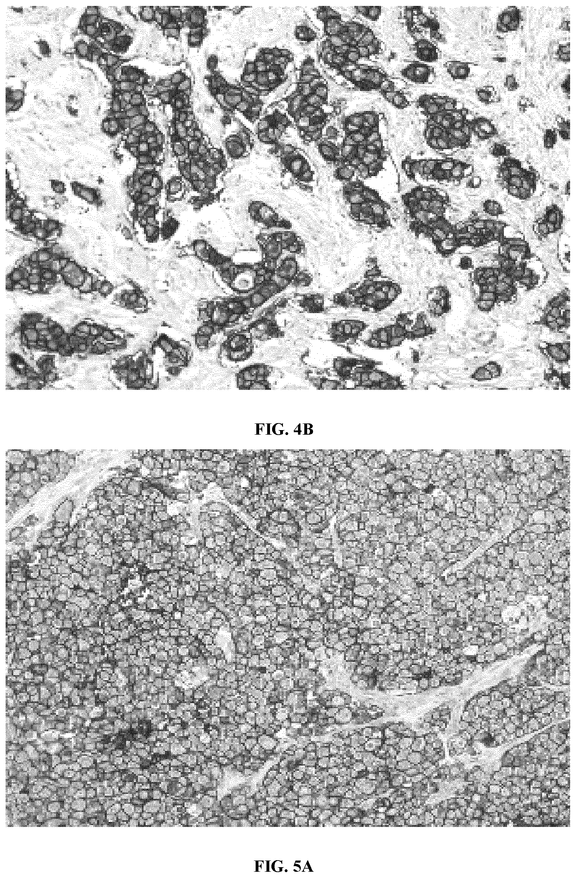

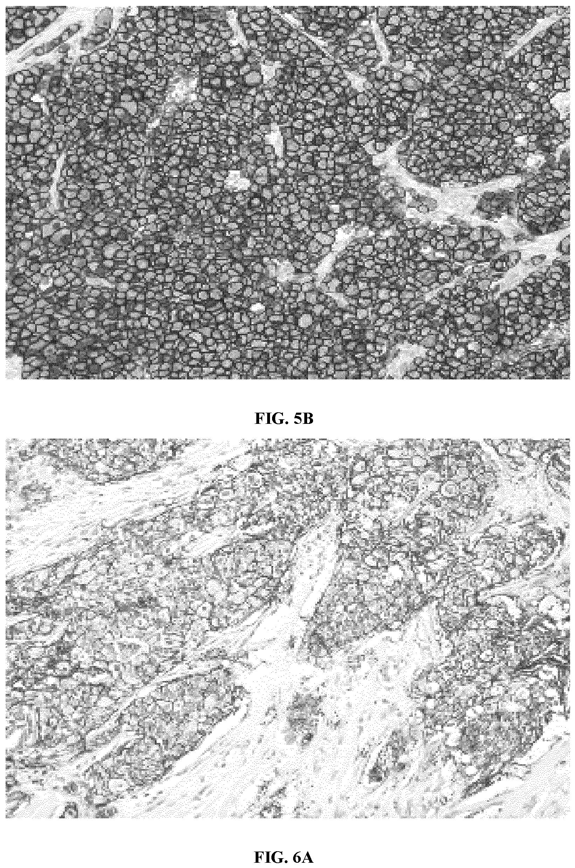

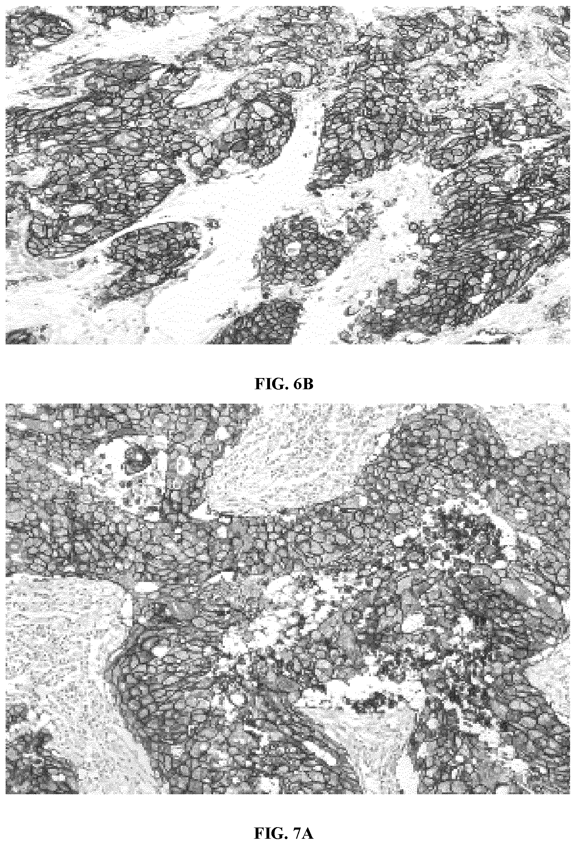

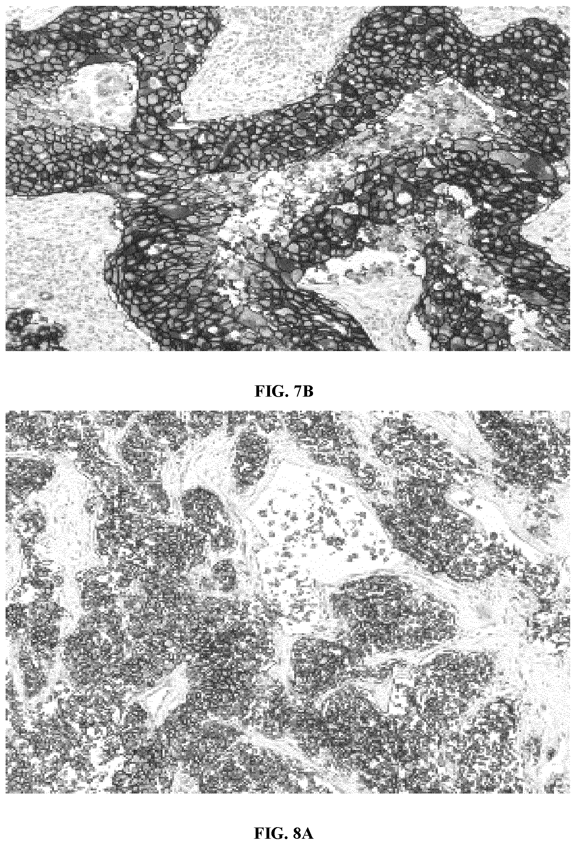

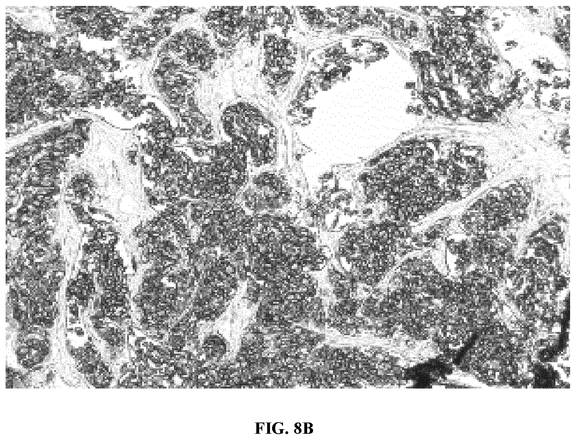

Then observed under the microscope and pictures were taken, with the objective lens 10×, the eyepiece 20×, and magnification 200 times. The staining results are shown in Figures 2-8, where Figures 2A, 3A, 4A, 5A, 6A, 7A, and 8A show the results of immunohistochemical staining with RC48M antibody as the primary antibody, and Figures 2B, 3B, 4B, 5B, 6B, 7B, and 8B show the results of immunohistochemical staining with 4B5 antibody as the primary antibody. The RC48M antibody and 4B5 antibody had similar staining in the cases of serial numbers 2, 3, 4, 5, 6, and 7, but showed a difference in the case of serial number 1, where RC48M staining was negative and 4B5 staining was positive. The above staining results were interpreted according to the rules in the "Guideline for HER2 Detection of Breast carcinoma (2014 Edition)". See Table 7 for the interpretation results. The staining results of the RC48M antibody and the 4B5 antibody were 3+ in the cases of serial numbers 3, 4, 5, and 6; were 2+ in the case of serial number 2; showed a difference of 1 grade in the case of serial number 7; and showed a difference of negative and 3+ in the case of serial number 1. According to the clinical trial drug efficacy information corresponding to the cases represented by each serial number, the disease of the case represented by serial number 1 continued to progress after clinical medication. That is, for the patient who was positive for the detection, the use of targeted drugs was ineffective, which means that the case of serial number 1 had false positives when detected with 4B5 antibody. The results show that the RC48M antibody can effectively reduce the false positive problems in the detection caused by mutations in the extracellular region of Her2, so that it can more accurately determine the follow-up treatment regimen for such patients and avoid ineffective targeted drug treatment, and thus greatly saves the ineffective treatment investment for such patients and provide more accurate detection reference for the subsequent selection of other treatment regimens.

**Table 7 Detection results and clinical efficacy**

| Serial Number | RC48M | 4B5 | Clinical Efficacy |
|---|---|---|---|
| 1 | 0 | 3+ | PD |
| 2 | 2+ | 2+ | PD |
| 3 | 3+ | 3+ | PR |
| 4 | 3+ | 3+ | SD |
| 5 | 3+ | 3+ | PR |
| 6 | 3+ | 3+ | PR |
| 7 | 2+ | 3+ | SD |

| | | | |
|---|---|---|---|
| Note: PD stands for progressive disease; PR stands for partial remission; and SD stands for stable disease. | | | |

The present disclosure has been exemplified by various specific embodiments. However, those of ordinary skill in the art can understand that the present disclosure is not limited to specific embodiments. Those of ordinary skill in the art can make various modifications and variations within the scope of the present disclosure, and various technical features mentioned in various places of this specification can be combined with each other without departing from the spirit and scope of the present disclosure. Such modifications and variations are all within the scope of the present disclosure.

## Claims

1. An antibody for companion diagnostic immunohistochemical (IHC) detection of human epidermal growth factor receptor 2 (Her2), wherein the antibody specifically binds to extracellular domain IV region of Her2 protein and has the capability of effectively detecting IHC samples without antigen or epitope retrieval treatment.

2. The antibody according to claim 1, wherein Fc fragment of the antibody is Fc fragment of a non-human mammal antibody, preferably a murine-derived Fc fragment or a rabbit-derived Fc fragment.

3. The antibody according to claim 1 or 2, wherein the antibody competitively binds to the same or a similar epitope with a CDR-defined antibody, and the CDRs 1-3 of a heavy chain variable region of the CDR-defined antibody have the amino acid sequences shown in SEQ ID NOs: 1-3, respectively; and the CDRs 1-3 of a light chain variable region of the CDR-defined antibody have the amino acid sequences shown in SEQ ID NOs: 4-6, respectively.

4. The antibody according to any one of claims 1 to 3, wherein, in the antibody:
(i) the CDR 1 of the heavy chain variable region has an amino acid sequence shown in SEQ ID NO: 1 or an amino acid sequence obtained after 1 or 2 amino acids substitution on SEQ ID NO: 1; or/and the CDR 2 of the heavy chain variable region has an amino acid sequence shown in SEQ ID NO: 2 or an amino acid sequence obtained after 1, 2, 3, 4 or 5 amino acids substitution on SEQ ID NO: 2; or/and the CDR 3 of the heavy chain variable region has an amino acid sequence shown in SEQ ID NO: 3 or an amino acid sequence obtained after 1, 2 or 3 amino acids substitution on SEQ ID NO : 3; and/or
(ii) the CDR 1 of the light chain variable region has an amino acid sequence shown in SEQ ID NO: 4 or an amino acid sequence obtained after 1, 2, 3, or 4 amino acids substitution on SEQ ID NO: 4; or/and the CDR 2 of the light chain variable region has an amino acid sequence shown in SEQ ID NO: 5 or an amino acid sequence obtained after 1 or 2 amino acids substitution on SEQ ID NO: 5; or/and the CDR 3 of the light chain variable region has an amino acid sequence shown in SEQ ID NO: 6 or an amino acid sequence obtained after 1 or 2 amino acids substitution on SEQ ID NO: 6;
and/or
(iii) the immunoglobulin Fc fragment is a murine-derived IgG Fc fragment, or further is a murine-derived IgG1 Fc fragment.

5. The antibody according to claim 4, wherein, in the antibody:
(i) the CDRs 1-3 of the heavy chain variable region have an amino acid sequences shown in SEQ ID NOs: 1-3, respectively; and
(ii) the CDRs 1-3 of the light chain variable region have an amino acid sequences shown in SEQ ID NOs: 4-6, respectively.

6. The antibody according to any one of claims 1-5, wherein
(i) the heavy chain of the antibody comprises an amino acid sequence shown in SEQ ID NO: 7 or an amino acid sequence having at least 80% sequence identity thereto; and/or
(ii) the light chain of the antibody comprises an amino acid sequence shown in SEQ ID NO: 8 or an amino acid sequence having at least 80% sequence identity thereto; and
(iii) the CDRs 1-3 of the heavy chain variable region of the antibody have an amino acid sequences shown in SEQ ID NOs: 1-3, respectively; the CDRs 1-3 of the light chain variable region of the antibody have an amino acid sequences shown in SEQ ID NOs: 4-6, respectively.

7. The antibody according to claim 6, wherein
(i) the heavy chain of the antibody has an amino acid sequence shown in SEQ ID NO: 7; and
(ii) the light chain of the antibody has an amino acid sequence shown in SEQ ID NO:8.

8. A nucleic acid molecule encoding the antibody according to any one of claims 1 to 7.

9. A vector comprising the nucleic acid molecule according to claim 7.

10. Use of the antibody according to any one of claims 1 to 7 in the manufacture of a Her2 companion diagnostic immunohistochemical detection product, wherein the immunohistochemical detection product has a function of not requiring a step of the antigen or epitope retrieval during the immunohistochemical detection; preferably, the Her2 companion diagnostic immunohistochemical detection product is a matching one used before RC48-ADC targeted drug therapy; more preferably, the indications targeted by the Her2 companion diagnostic immunohistochemical detection product are Her2-related cancers, further preferably breast carcinoma, gastric carcinoma, gastroesophageal carcinoma, oesophageal carcinoma, ovarian carcinoma, endometrial carcinoma, lung carcinoma, urothelial carcinoma, or bladder carcinoma.

11. A Her2 companion diagnostic immunohistochemical detection kit comprising the antibody according to any one of claims 1-7, wherein the kit has the function of not requiring a step of the antigen or epitope retrieval during the immunohistochemical detection; preferably, the Her2 companion diagnostic immunohistochemical detection kit is a matching one used before RC48-ADC targeted drug therapy; more preferably, the indications targeted by the Her2 companion diagnostic immunohistochemical detection kit are Her2-related cancers, further preferably breast carcinoma, gastric carcinoma, gastroesophageal carcinoma, oesophageal carcinoma, ovarian carcinoma, endometrial carcinoma, lung carcinoma, urothelial carcinoma, or bladder carcinoma.


SEQUENCE LISTING

<110>  ÈÙ²ýÉúÎïÖÆÒ©£¨ÑÌÌ¨£©ÓÐÏÞ¹«Ë¾

<120>  Ò»ÖÖHer2°éËæÕï¶ÏÃâÒß×é»¯¼ì²â¿¹Ìå¼°ÆäÓ¦ÓÃ

<130>  2019

<160>  8

<170>  PatentIn version 3.3

<210>  1
<211>  5
<212>  PRT
<213>  ÈË¹¤ÐòÁÐ

<400>  1

Asp Tyr Tyr Ile His
1               5

<210>  2
<211>  17
<212>  PRT
<213>  ÈË¹¤ÐòÁÐ

<400>  2

Arg Val Asn Pro Asp His Gly Asp Ser Tyr Tyr Asn Gln Lys Phe Lys
1               5                   10                  15

Asp

<210>  3
<211>  9
<212>  PRT
<213>  ÈË¹¤ÐòÁÐ

<400>  3

Ala Arg Asn Tyr Leu Phe Asp His Trp
1               5

<210>  4
<211>  11
<212>  PRT
<213>  ÈË¹¤ÐòÁÐ

<400>  4

Lys Ala Ser Gln Asp Val Gly Thr Ala Val Ala
1               5                   10

<210>  5
<211>  7
<212>  PRT

<213>  ÈË¹¤ÐòÁÐ

<400>  5

Trp Ala Ser Ile Arg His Thr
1               5


<210>  6
<211>  7
<212>  PRT
<213>  ÈË¹¤ÐòÁÐ

<400>  6

His Gln Phe Ala Thr Tyr Thr
1               5


<210>  7
<211>  439
<212>  PRT
<213>  ÈË¹¤ÐòÁÐ

<400>  7

Glu Val Gln Leu Val Gln Ser Gly Ala Glu Val Lys Lys Pro Gly Ala
1               5                   10                  15

Thr Val Lys Ile Ser Cys Lys Val Ser Gly Tyr Thr Phe Thr Asp Tyr
            20                  25                  30

Tyr Ile His Trp Val Gln Gln Ala Pro Gly Lys Gly Leu Glu Trp Met
        35                  40                  45

Gly Arg Val Asn Pro Asp His Gly Asp Ser Tyr Tyr Asn Gln Lys Phe
    50                  55                  60

Lys Asp Lys Ala Thr Ile Thr Ala Asp Lys Ser Thr Asp Thr Ala Tyr
65                  70                  75                  80

Met Glu Leu Ser Ser Leu Arg Ser Glu Asp Thr Ala Val Tyr Phe Cys
                85                  90                  95

Ala Arg Asn Tyr Leu Phe Asp His Trp Gly Gln Gly Thr Leu Val Thr
            100                 105                 110

Val Ser Ser Ala Lys Thr Thr Pro Pro Ser Val Tyr Pro Leu Ala Pro
        115                 120                 125

Gly Ser Ala Ala Gln Thr Asn Ser Met Val Thr Leu Gly Cys Leu Val
    130                 135                 140

Lys Gly Tyr Phe Pro Glu Pro Val Thr Val Thr Trp Asn Ser Gly Ser

145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Pro Ser Ser Thr Trp Pro Ser
            180                 185                 190

Glu Thr Val Thr Cys Asn Val Ala His Pro Ala Ser Ser Thr Lys Val
        195                 200                 205

Asp Lys Lys Ile Val Pro Arg Asp Cys Gly Cys Lys Pro Cys Ile Cys
    210                 215                 220

Thr Val Pro Glu Val Ser Ser Val Phe Ile Phe Pro Pro Lys Pro Lys
225                 230                 235                 240

Asp Val Leu Thr Ile Thr Leu Thr Pro Lys Val Thr Cys Val Val Val
                245                 250                 255

Asp Ile Ser Lys Asp Asp Pro Glu Val Gln Phe Ser Trp Phe Val Asp
            260                 265                 270

Asp Val Glu Val His Thr Ala Gln Thr Gln Pro Arg Glu Glu Gln Phe
        275                 280                 285

Asn Ser Thr Phe Arg Ser Val Ser Glu Leu Pro Ile Met His Gln Asp
    290                 295                 300

Trp Leu Asn Gly Lys Glu Phe Lys Cys Arg Val Asn Ser Ala Ala Phe
305                 310                 315                 320

Pro Ala Pro Ile Glu Lys Thr Ile Ser Lys Thr Lys Gly Arg Pro Lys
                325                 330                 335

Ala Pro Gln Val Tyr Thr Ile Pro Pro Pro Lys Glu Gln Met Ala Lys
            340                 345                 350

Asp Lys Val Ser Leu Thr Cys Met Ile Thr Asp Phe Phe Pro Glu Asp
        355                 360                 365

Ile Thr Val Glu Trp Gln Trp Asn Gly Gln Pro Ala Glu Asn Tyr Lys
    370                 375                 380

Asn Thr Gln Pro Ile Met Asp Thr Asp Gly Ser Tyr Phe Val Tyr Ser
385                 390                 395                 400

Lys Leu Asn Val Gln Lys Ser Asn Trp Glu Ala Gly Asn Thr Phe Thr
                405                 410                 415

Cys Ser Val Leu His Glu Gly Leu His Asn His His Thr Glu Lys Ser
            420                 425                 430

Leu Ser His Ser Pro Gly Ile
        435

<210> 8
<211> 212
<212> PRT
<213> ÈË¹¤ÐòÁÐ

<400> 8

Asp Ile Gln Met Thr Gln Ser Pro Ser Ser Val Ser Ala Ser Val Gly
1               5                   10                  15

Asp Arg Val Thr Ile Thr Cys Lys Ala Ser Gln Asp Val Gly Thr Ala
            20                  25                  30

Val Ala Trp Tyr Gln Gln Lys Pro Gly Lys Ala Pro Lys Leu Leu Ile
        35                  40                  45

Tyr Trp Ala Ser Ile Arg His Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Phe Ala Thr Tyr Thr Phe
                85                  90                  95

Gly Gly Gly Thr Lys Val Glu Ile Lys Arg Thr Val Ala Ala Pro Ser
            100                 105                 110

Val Phe Ile Phe Pro Pro Ser Asp Glu Gln Leu Lys Ser Gly Thr Ala
        115                 120                 125

Ser Val Val Cys Leu Leu Asn Asn Phe Tyr Pro Arg Glu Ala Lys Val
    130                 135                 140

Gln Trp Lys Val Asp Asn Ala Leu Gln Ser Gly Asn Ser Gln Glu Ser
145                 150                 155                 160

Val Thr Glu Gln Asp Ser Lys Asp Ser Thr Tyr Ser Leu Ser Ser Thr
                165                 170                 175

Leu Thr Leu Ser Lys Ala Asp Tyr Glu Lys His Lys Val Tyr Ala Cys
            180                 185                 190

Glu Val Thr His Gln Gly Leu Ser Ser Pro Val Thr Lys Ser Phe Asn
        195                 200                 205

Arg Gly Glu Cys
    210